# EUROPEAN PATENT SPECIFICATION

(11) **EP 4 174 487 B1**
(45) Date of publication and mention of the grant of the patent: **24.07.2024**
(21) Application number: 22199793.5
(22) Date of filing: 05.10.2022
(51) Int. Cl.: G01N 33/00, G06N 20/00, G06Q 10/087, F25D 17/04

(54) **ODOR DETECTION METHOD FOR REFRIGERATION APPLIANCE AND REFRIGERATION APPLIANCE**
GERUCHSERKENNUNGSVERFAHREN FÜR EIN KÜHLGERÄT UND KÜHLGERÄT
PROCÉDÉ DE DÉTECTION D'ODEUR POUR APPAREIL DE RÉFRIGÉRATION ET APPAREIL DE RÉFRIGÉRATION

(30) Priority: 01.11.2021 CN 202111282701
(43) Date of publication of application: 03.05.2023
(73) Proprietor: BSH Hausgeräte GmbH, 81739 München (DE)
(72) Inventor: Qu, Dean, Nanjing, 210046 (CN); Ju, Wangkou, Nanjing, 210046 (CN)

(56) References cited:
- WO-A1-2020/185101
- CAYA MEO VINCENT C ET AL: "Monitoring and Detection of Fruits and Vegetables Spoilage in the Refrigerator using Electronic Nose Based on Principal Component Analysis", 2019 IEEE 11TH INTERNATIONAL CONFERENCE ON HUMANOID, NANOTECHNOLOGY, INFORMATION TECHNOLOGY, COMMUNICATION AND CONTROL, ENVIRONMENT, AND MANAGEMENT ( HNICEM ), IEEE, 29 November 2019 (2019-11-29), pages 1 - 6, XP033760657, DOI: 10.1109/HNICEM48295.2019.9072715
- WANG MIN ET AL: "Real-time assessment of food freshness in refrigerators based on a miniaturized electronic nose", ANALYTICAL METHODS, vol. 10, no. 39, 20 July 2018 (2018-07-20), GB, pages 4741 - 4749, XP093022235, ISSN: 1759-9660, DOI: 10.1039/C8AY01242C

## Description

### TECHNICAL FIELD

Embodiments of the present invention relate to the field of refrigeration appliance technologies, and specifically, to an odor detection method for a refrigeration appliance and a refrigeration appliance.

### BACKGROUND

With the popularization of refrigeration appliances in people's daily life, an increasing number of people begin to use refrigeration appliances, such as refrigerators, to store perishable food such as vegetables, fish, and meat. Food placed in a refrigeration appliance inevitably produces peculiar smells. If the peculiar smells cannot be eliminated in time, the user experience will be adversely affected. However, peculiar smells in the refrigeration appliance cannot be accurately detected by using the existing technology, let alone eliminate the peculiar smells in time.

CAYA MEO VINCENT C ET AL: "Monitoring and Detection of Fruits and Vegetables Spoilage in the Refrigerator using Electronic Nose Based on Principal Component Analysis", 2019 IEEE 11TH INTERNATIONAL CONFERENCE ON HUMANOID, NANOTECHNOLOGY, INFORMATION TECHNOLOGY, COMMUNICATION AND CONTROL, ENVIRONMENT, AND MANAGEMENT ( HNICEM ), IEEE, 29 November 2019 (2019-11-29), pages 1-6, XP033760657, DOI: 10.1109/HNICEM48295.2019.9072715 describes a method wherein gas data from a refrigerator are processed by a machine learning model and odour attributes are determined.

### SUMMARY

An objective of embodiments of the present invention is to provide an improved refrigeration appliance and an odor detection method for a refrigeration appliance.

Therefore, the embodiments of the present invention provide an odor detection method for a refrigeration appliance, including: obtaining gas data in the refrigeration appliance; inputting the gas data into a plurality of first preset machine learning models respectively, and obtaining first prediction results of the first preset machine learning models, where the first prediction result includes an odor attribute in the refrigeration appliance and a corresponding probability, and the first preset machine learning model is trained based on gas data of a single type of food under a plurality of odor attributes; determining prediction confidences of the first preset machine learning models according to differences between a plurality of first prediction results; and determining; if the prediction confidences are high, an odor attribute corresponding to a probability maximum in the plurality of first prediction results as a current odor attribute in the refrigeration appliance.

Adopting this implementation can improve the accuracy of odor detection, which is conducive to timely detection and elimination of a peculiar smell, thereby better maintaining the air in the refrigeration appliance fresh. Specifically, gas data associated with a single type of food may be regarded as simple data, and the first preset machine learning model constructed based on the simple data has high accuracy in predicting an odor attribute of a specific type of food. Based on this, on the premise that the prediction confidence of the first preset machine learning model satisfies an expectation, determining the current odor attribute in the refrigeration appliance according to the first preset result is conducive to obtaining a highly accurate odor detection result.

Optionally, the odor detection method further includes: inputting, if the prediction confidences are low, the gas data into at least one second preset machine learning model and obtaining a second prediction result, where the second prediction result includes an odor attribute in the refrigeration appliance and a corresponding probability, and the second preset machine learning model is trained based on gas data of mixed types of foods under a plurality of odor attributes; and determining the current odor attribute in the refrigeration appliance according to the first prediction results and the second prediction result. The gas data associated with the mixed types of foods may be regarded as complex data. The second preset machine learning model constructed based on the complex data has high adaptability, and in a practical application, can reliably predict an odor attribute of a gas produced by a plurality of types of foods together. Based on this, a comprehensive data model trained based on the odor data associated with the mixed types of foods is used as an auxiliary means. When confidences of the first preset machine learning models trained based on a single type of food are low, a final prediction result is comprehensively determined with the assistance of a prediction result of the second preset machine learning model, which is conducive to improving the accuracy of the prediction result.

Optionally, the determining the current odor attribute in the refrigeration appliance according to the first prediction results and the second prediction result includes: determining an odor attribute corresponding to a probability maximum in the plurality of first prediction results and the second prediction results as the current odor attribute in the refrigeration appliance. Therefore, the prediction accuracy is improved through comprehensive discrimination of a plurality models.

Optionally, the determining prediction confidences of the first preset machine learning models according to differences between a plurality of first prediction results includes: determining that the prediction confidences are low if in the plurality of first prediction results, a deviation between the probability maximum and a secondary probability maximum is less than a first preset threshold, and the probability maximum and the secondary probability maximum respectively correspond to different odor attributes; and determining that the prediction confidences are high if in the plurality of first prediction results, the deviation between the probability maximum and the secondary probability maximum is greater than the first preset threshold. When the probability maximum and the secondary probability maximum correspond to different odor attributes respectively, it indicates that there are large deviations in predictions of the first preset machine learning models that output the two prediction results. When the foregoing case occurs, the prediction confidences of the first prediction results may be considered as low, and then the second preset machine learning model is introduced to assist in discrimination, to ensure the accuracy of the final prediction result.

Optionally, the gas data includes concentration information of at least one gas component in the refrigeration appliance.

Optionally, the first preset machine learning model includes a plurality of sub-models, the different sub-models are constructed by using different algorithms, and the first prediction result of the first preset machine learning model is an average of respective prediction results of the plurality of sub-models; and/or the second preset machine learning model includes a plurality of sub-models, different sub-models are constructed by using different algorithms, and the second prediction result of the second preset machine learning model is an average of respective prediction results of the plurality of sub-models. Therefore, for a single first preset machine learning model and/or a single second preset machine learning model, an output result of the model is also a product of integrating prediction results of a plurality of sub-models, which is conducive to further improving the accuracy of the prediction result of the model.

Optionally, training data of the first preset machine learning model is selected from a single training set, and training data of the second preset machine learning model is selected from a plurality of training sets, where the training set has a one-to-one correspondence with a type of food. Therefore, different training sets or different combinations of training sets are used to train a plurality of first preset machine learning models and second preset machine learning models, so that different models are sensitive to odors produced by different types of foods or combinations of different types of foods in odor attribute stages. In this way, in a practical application, a proper model can be used to perform prediction in any odor occasion.

Optionally, before the determining prediction confidences of the first preset machine learning models according to differences between a plurality of first prediction results, the method further includes: determining whether all the first prediction results are higher than a second preset threshold; and obtaining, if a determination result indicates that any first prediction result is lower than the second preset threshold, updated gas data, and obtaining updated first prediction results based on the updated gas data. The second preset threshold can be used to represent reliability of a single first prediction result. When the reliability of the first prediction result is low, the gas data is re-acquired for the first preset machine learning model, and prediction is performed. Therefore, controlling the reliability of a plurality of first prediction results that serve as a data basis is conducive to improving the accuracy of the final prediction result.

Optionally, the odor attribute includes at least one of the following: pleasant or unpleasant.

Therefore, the embodiments of the present invention further provide a refrigeration appliance, including: a gas detector, configured to acquire gas data in the refrigeration appliance; a control module, communicating with the gas detector to receive the gas data, where the control module is configured to perform the foregoing odor detection method and generate a control instruction according to a current odor attribute in the refrigeration appliance; and a deodorization module, configured to adjust an operation parameter according to the control instruction in response to receiving the control instruction.

Therefore, when a peculiar smell is detected based on the odor detection method provided in this implementation, the operation of the deodorization module is actively controlled to eliminate the peculiar smell in time.

Optionally, the refrigeration appliance further includes: a communication module, where the control module communicates with the gas detector through the communication module, and/or the control module communicates with the deodorization module through the communication module.

### BRIEF DESCRIPTION OF THE DRAWINGS

FIG. 1 is a flowchart of an odor detection method for a refrigeration appliance according to an embodiment of the present invention;
FIG. 2 is a flowchart of a specific implementation of step S101 in FIG. 1;
FIG. 3 is a flowchart of a training process of a first preset machine learning model according to an embodiment of the present invention;
FIG. 4 is a flowchart of a specific implementation of step S103 in FIG. 1;
FIG. 5 is a schematic diagram of a typical application scenario of a refrigeration appliance according to an embodiment of the present invention; and
FIG. 6 is a logical block diagram when a control module in FIG. 5 executes an odor detection method shown in FIG. 1.

In the figures:
1-refrigeration appliance; 10-compartment; 11-gas detector; 12-control module; 13-deodorization module; and 14-communication module.

### DETAILED DESCRIPTION

To make the foregoing objectives, features, and advantages of the present invention more comprehensible, specific embodiments of the present invention are described below in detail with reference to the accompanying drawings.

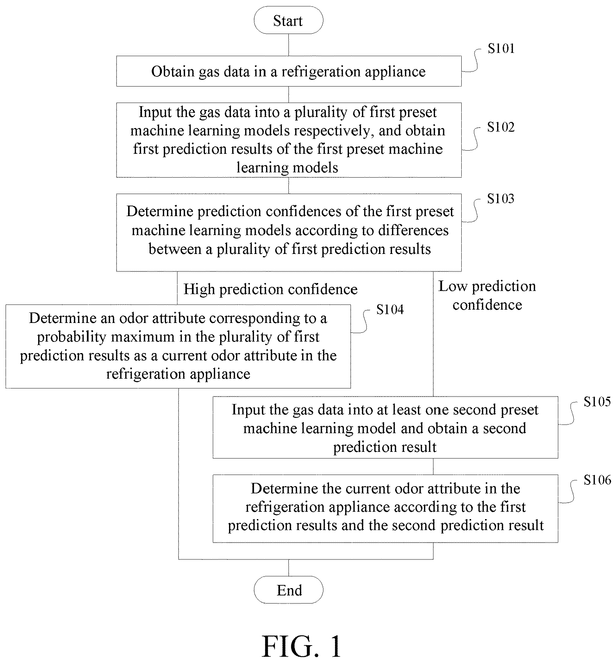

FIG. 1 is a flowchart of an odor detection method for a refrigeration appliance according to an embodiment of the present invention.

This embodiment can be applied to an odor monitoring scenario in a refrigeration appliance, such as monitoring whether there is a peculiar smell in the refrigeration appliance, and monitoring a real-time odor attribute in the refrigeration appliance. The odor attribute may include five categories such as extremely pleasant, pleasant, neutral, unpleasant, and extremely unpleasant. In a practical application, classification standards of the odor attribute and a number of classified categories can be adjusted according to needs. The food stored in the refrigeration appliance may belong to one or more types. In this embodiment, an odor attribute of an odor generated by a specific type of food in the refrigeration appliance can be specifically detected, and an odor attribute of a mixed odor generated by a variety of foods in the refrigeration appliance can also be detected as a whole on a macroscopic level.

Specifically, referring to FIG. 1, an odor detection method for a refrigeration appliance according to this embodiment may include the following steps:
Step S101. Obtain gas data in the refrigeration appliance.
Step S102. Input the gas data into a plurality of first preset machine learning models respectively, and obtain first prediction results of the first preset machine learning models, where the first prediction result includes an odor attribute in the refrigeration appliance and a corresponding probability, and the first preset machine learning model is trained based on gas data of a single type of food under a plurality of odor attributes.
Step S103. Determine prediction confidences of the first preset machine learning model according to differences between a plurality of first prediction results.
Step S104. Determine, if the prediction confidences are high, an odor attribute corresponding to a probability maximum in the plurality of first prediction results as a current odor attribute in the refrigeration appliance.

Further, the gas data may include concentration information of at least one gas component in the refrigeration appliance. Different types of foods may produce different types of gas components under different odor attributes. An intuitive feeling for a user is that different odors are smelled. For example, the foods may include, by category, meat, nuts, vegetables and the like. The odor attribute may include extremely pleasant, pleasant, neutral, unpleasant, and extremely unpleasant. Evaluation standards for odor attributes may be determined according to sensitivity of the user to the odor, and may also be determined according to relevant industry standards.

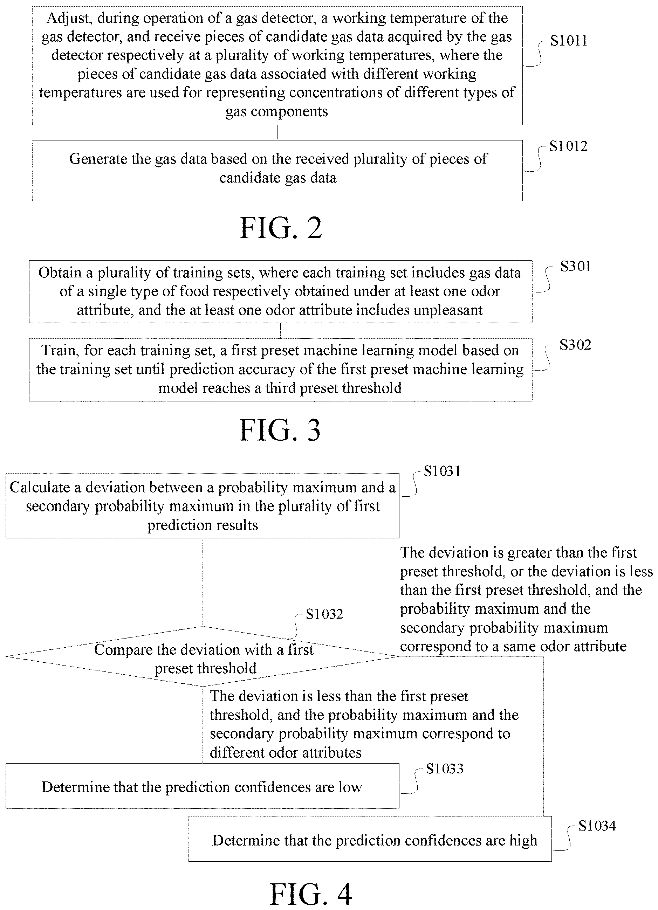

In a specific implementation, the gas data may be acquired by a gas detector arranged in the refrigeration appliance. Correspondingly, referring to FIG. 2, step S101 may include the following steps:
Step S1011. Adjust, during operation of the gas detector, a working temperature of the gas detector, and receive pieces of candidate gas data acquired by the gas detector respectively at a plurality of working temperatures, where the pieces of candidate gas data associated with different working temperatures are used for representing concentrations of different types of gas components.
Step S1012. Generate the gas data based on the received plurality of pieces of candidate gas data.

Therefore, the working temperature of the gas detector is adjusted within a specific temperature range to enrich the diversity of output data of the gas detector, so as to detect various gas components.

Specifically, the gas detector may include a volatile organic compound (VOC) sensor. The VOC sensor reacts violently to specific types of gases at different temperatures, and this characteristic is used to enable the gas detector used in the implementation to detect various gas components within a specific temperature range. For example, at a specific working temperature, the VOC sensor reacts with a specific type of gas component in the air and outputs a resistance value. The resistance value can be used to represent a concentration of the specific type of gas component.

Further, step S1011 may include the following step: adjusting, during the operation of the gas detector, the working temperature of the gas detector according to a candidate value selected from a preset working temperature set, where the preset working temperature set includes a plurality of candidate values of the working temperature. Properly designing the preset working temperature set enables the gas detector to accurately detect a variety of gas components that need to be detected.

A candidate value in the preset working temperature set may be determined according to the sensitivity of the gas detector to a gas component when the gas detector works at the candidate value. For example, a main gas component produced by various foods under odor attributes may be determined through pre-experiments. With the gas component as a target, a working temperature of the VOC sensor is adjusted to test out a working temperature at which the VOC sensor has the highest sensitivity (namely, sensitiveness) to such a gas component and a time required to detect the gas component. A working temperature at which the sensitivity is the highest is added to the preset working temperature set as a candidate value that may be selected when the implementation is implemented. Therefore, the gas detector can detect a specific type of gas component at a working temperature at which the gas detector is most sensitive to such a gas component, thereby ensuring the precision of gas data, which is conducive to ensuring the accuracy of a final prediction result.

Further, the candidate value selected from the preset working temperature set may be selected from the preset working temperature set in ascending, descending, or random order. Correspondingly, the working temperature of the gas detector continuously rises or falls, or fluctuates, so that concentrations of the gas component associated with candidate values are obtained.

For example, when step S1011 is performed, a candidate value with the smallest value may be selected from the preset working temperature set as an initial working temperature of the VOC sensor. After candidate gas data is acquired at the initial working temperature, the working temperature of the VOC sensor is gradually increased until the candidate gas data is acquired under a candidate value with the largest value in the preset working temperature set.

When the VOC sensor performs gas detection in a manner of continuous heating or cooling, the working temperature of the VOC sensor does not fluctuate violently in a short time, which is conducive to prolonging a service life of a device. When the VOC sensor randomly selects a candidate value from the preset working temperature set, there is a greater probability to quickly switch to a working temperature at which the VOC sensor is sensitive to a gas component produced under the current odor attributes, which is conducive to a rapid response of the VOC sensor.

Further, the preset working temperature set may be traversed for at least one round during the operation of the gas detector. At least one set of data is obtained through cyclic acquisition, so as to expand the amount of data inputted into the first preset machine learning model, which is conducive to improving the accuracy of the prediction result. For example, when step S1011 is performed, the preset working temperature set is repeatedly traversed to obtain concentrations of a plurality of groups of different types of gas components, and a plurality of concentrations of a same type of gas component are averaged, so as to integrate a plurality of sets of data into a set of gas data and input the set of gas data into first preset machine learning models. Therefore, impact of a single detection error on the prediction result is eliminated by obtaining an average through a plurality of detections.

Further, a time interval during which the VOC sensor switches from a current working temperature to a next working temperature may be determined according to a time required to detect a corresponding gas component at the current working temperature. The required time may be preset through experiments.

Further, the gas data may be generated based on all pieces of candidate gas data received after the preset working temperature set is traversed for at least one round, so that the comprehensiveness of the gas data can be ensured, and the gas data may include concentration information of all types of gas components that may be present in the refrigeration appliance within a period of time. For example, when step S1012 is performed, after the preset working temperature set is traversed for one round, all the obtained pieces of candidate gas data are inputted as gas data into the first preset machine learning models. In another example, after the preset working temperature set is traversed for a plurality of rounds, a plurality of pieces of candidate gas data obtained at the same working temperature are integrated into a single piece of candidate gas data, and the integrated candidate gas data at all working temperatures is inputted as final gas data into the first preset machine learning models.

Alternatively, a quantity of pieces of candidate gas data for generating the gas data may be less than a quantity of candidate values in the preset working temperature set, so that a response speed of the gas detector is higher and the gas data can be generated more quickly. For example, when step S1012 is performed, after being obtained, one or more pieces of candidate gas data are inputted as gas data into the first preset machine learning models, so as to obtain the prediction result faster.

In a specific implementation, the candidate values in the preset working temperature set may be used to detect a gas component that may be generated when a variety of foods are stored together in the refrigeration appliance. For example, a gas generated when a variety of foods, such as meat for cooking and vegetables, are stored separately and together is acquired, and gas components are analyzed. Appropriate candidate values are determined respectively according to the various gas components obtained through analysis, to finally generate the preset working temperature set. Correspondingly, when step S1011 is performed, regardless of the type of food currently stored in the refrigeration appliance, a candidate value is selected from a single preset working temperature set as the working temperature of the VOC sensor.

In a variant, the preset working temperature set may be specifically designed for different types of foods. For example, a preset working temperature set may be constructed for a working temperature at which the VOC sensor is sensitive to the gas component that may be generated when meat food is stored in the refrigeration appliance, and a preset working temperature set may be additionally constructed for a working temperature at which the VOC sensor is sensitive to a gas component generated when nut food is stored in the refrigeration appliance. Correspondingly, when step S1011 is performed, a corresponding preset working temperature set is retrieved according to the type of food currently stored in the refrigeration appliance, and a candidate value is selected from the preset working temperature set as the working temperature of the VOC sensor. When a variety of foods are stored in the refrigeration appliance at the same time, preset working temperature sets corresponding to the foods are retrieved respectively for detection. For example, the type of food currently stored in the refrigeration appliance may be obtained from user input information, or may be determined through active detection of a sensing device such as an image sensor.

In a specific implementation, training data of the first preset machine learning model may be selected from a single training set. The training set has a one-to-one correspondence with a type of food. Each training set includes gas data of a single type of food respectively obtained under at least one odor attribute, and the at least one odor attribute includes unpleasant.

Correspondingly, referring to FIG. 3, a training process of the first preset machine learning model according to this embodiment may include the following steps:
Step S301. Obtain a plurality of training sets, where each training set includes gas data of a single type of food respectively obtained under at least one odor attribute, and the at least one odor attribute includes unpleasant.
Step S302. Train, for each training set, the first preset machine learning model based on the training set until prediction accuracy of the first preset machine learning model reaches a third preset threshold.

Specifically, the first preset machine learning model may be selected from a plurality of candidate models, where different candidate models are constructed by using different algorithms.

For example, in step S302, the candidate models may be trained respectively based on the training sets until prediction accuracy of the candidate models reaches the third preset threshold. Then, a candidate model with the highest prediction accuracy in a plurality of candidate models obtained through training is selected as the first preset machine learning model. Therefore, using the candidate model with the best prediction performance as a first preset machine learning model for final use is conducive to improving the recognition accuracy of the odor attribute in a practical application stage.

In another example, in step S302, the candidate models may be trained respectively based on the training sets, and a candidate model of which the prediction accuracy first reaches the third preset threshold in the candidate models is determined as the first preset machine learning model.

A specific value of the third preset threshold may be set according to a user needs, for example, the value may range from 85% to 99%. The third preset threshold can be used to measure a tolerance of the user for a prediction error rate of a model. Preset thresholds of different candidate models may be the same or different.

The algorithm used to construct a candidate model may be selected from: multilayer perceptron (MLP), a random forest algorithm, a logistic regression algorithm, a decision tree, an extreme gradient boosting decision tree (XGBoost), a k-nearest neighbor (KNN) classification algorithm, and the like.

The prediction accuracy of the candidate model may be obtained through testing with a test set after the model is preliminarily trained based on the training set. For a process of obtaining the test set, reference may be made to a generation process of the training set. The difference is that the test set is not inputted to the candidate model in a model construction stage.

In a typical application scenario, different types of foods may be consciously placed from producing a pleasant odor to producing an extremely unpleasant odor, and a gas generated during this time is acquired. Then, a gas analyzer is configured to analyze a gas component in the acquired gas, for example, analyze a main gas component that appears in the gas when an unpleasant odor is generated. A dynamic heating temperature of the VOC sensor is adjusted according to the analyzed gas component, so that the VOC sensor is more sensitive to the gas component. The adjusted VOC sensor is mounted at a proper position in the refrigeration appliance, and is prepared to acquire gas data.

Next, a list of foods is designed for odor detection experiments. The foods are placed respectively in the refrigeration appliance, and gas data in the refrigeration appliance during the placement is acquired based on the VOC sensor. In addition, calibration personnel are arranged to perform real-time calibration on site. For example, the calibration personnel can calibrate a current odor attribute of the gas in the refrigeration appliance as neutral when there is no unpleasant odor, and calibrate the current odor attribute of the gas in the refrigeration appliance as unpleasant when an unpleasant odor occurs for the first time.

Further, the unpleasant odor may be further subdivided into unpleasant and extremely unpleasant. A training set may be generated according to a real-time calibration result of the calibration personnel and the corresponding gas data.

All the odor detection experiments are performed at least three times, and data is acquired and calibrated during each experiment.

According to the acquired data, data of the last experiment is used as the test set, and the remaining data is divided into a training set and a validation set for generating the first preset machine learning model. For example, the first preset machine learning model using a specific algorithm is constructed based on the training set. Then a model parameter of the first preset machine learning model is verified and adjusted by using the validation set. Finally, the prediction accuracy of the first preset machine learning model is tested by using the test set.

In a specific implementation, the first preset machine learning model can predict an odor attribute and a corresponding probability with reference to the gas data and an operation parameter of the refrigeration appliance. Correspondingly, the training set may further include operation parameters of the refrigeration appliance when pieces of gas data are obtained, where the operation parameters may include a compartment temperature and a compartment humidity.

For example, a temperature and humidity acquisition device may be arranged in a compartment to acquire the compartment temperature and the compartment humidity. In another example, the operation parameter may be acquired by multiplexing the VOC sensor. In another example, the operation parameter may be directly obtained from a control module of the refrigeration appliance.

Correspondingly, step S102 may include the following step: inputting the gas data and the operation parameter synchronously obtained with the gas data into the first preset machine learning models and obtaining prediction results. Therefore, with reference to the operation parameter, the diversity of training samples is further expanded, which is conducive to optimizing a training effect of the first preset machine learning model and improving the prediction accuracy.

In view of the above, adopting this implementation can improve the accuracy of odor detection, which is conducive to timely detection and elimination of a peculiar smell, thereby better maintaining the air in the refrigeration appliance fresh. Specifically, gas data associated with a single type of food may be regarded as simple data, and the first preset machine learning model constructed based on the simple data has high accuracy in predicting an odor attribute of a specific type of food. Based on this, on the premise that the prediction confidence of the first preset machine learning model satisfies an expectation, determining the current odor attribute in the refrigeration appliance according to the first preset result is conducive to obtaining a highly accurate odor detection result.

Further, continuously referring to FIG. 1, the odor detection method according to this embodiment may further include the following steps:
Step S105. Input, if the prediction confidences are low, the gas data into at least one second preset machine learning model, and obtain a second prediction result, where the second prediction result includes an odor attribute in the refrigeration appliance and a corresponding probability, and the second preset machine learning model is trained based on gas data of mixed types of foods under a plurality of odor attributes.
Step S106. Determine the current odor attribute in the refrigeration appliance according to the first prediction results and the second prediction result.

Specifically, the first preset machine learning models may be constructed by adopting the same or different algorithms. Similarly, the second preset machine learning model may be constructed by adopting an algorithm the same or different from that of the first preset machine learning model, or a plurality of second preset machine learning models may also be constructed by adopting the same or different algorithms.

Further, a candidate model with the best prediction performance may be selected from a plurality of candidate models constructed based on the same training set and different algorithms as any one of the first preset machine learning models or the second preset machine learning model.

For a training process of the second preset machine learning model, reference may be made to the relevant description of training the first preset machine learning model in the embodiment shown in FIG. 3 above. The difference is that training data of the second preset machine learning model is selected from a plurality of training sets.

For example, the second preset machine learning model may be trained based on all the training sets. In another example, it is assumed that there are three training sets: a training set 1, a training set 2, and a training set 3, where one first preset machine learning model can be trained through each training set correspondingly. In addition, a second machine learning model 1 is trained based on the training set 1 and the training set 2, and a second preset machine learning model 2 is trained based on the training set 1, the training set 2, and the training set 3. Therefore, the diversity of training samples formed by a plurality of training sets enables the second preset machine learning model to also perform well in a complex environment and accurately identify different odor attributes of different foods.

The gas data associated with the mixed types of foods may be regarded as complex data. The second preset machine learning model constructed based on the complex data has high adaptability, and in a practical application, can reliably predict an odor attribute of a gas produced by a plurality of types of foods together. Based on this, a comprehensive data model trained based on the odor data associated with the mixed types of foods is used as an auxiliary means. When confidences of the first preset machine learning models trained based on a single type of food are low, a final prediction result is comprehensively determined with the assistance of a prediction result of the second preset machine learning model, which is conducive to improving the accuracy of the prediction result.

Therefore, different training sets or different combinations of training sets are used to train a plurality of first preset machine learning models and second preset machine learning models, so that different models are sensitive to odors produced by different types of foods or combinations of different types of foods in odor attribute stages. In this way, in a practical application, a proper model can be used to perform prediction in any odor occasion.

In a specific implementation, referring to FIG. 4, step S103 may include the following steps:
Step S1031. Calculate a deviation between a probability maximum and a secondary probability maximum in the plurality of first prediction results.
Step S1032. Compare the deviation with a first preset threshold.
Step S1033 is performed to determine that the prediction confidences are low if the deviation is less than the first preset threshold, and the probability maximum and the secondary probability maximum correspond to different odor attributes respectively.
Step S1034 is performed to determine that the prediction confidences are high if the deviation is greater than the first preset threshold, or the deviation is less than the first preset threshold and the two correspond to a same odor attribute.

Specifically, when the probability maximum and the secondary probability maximum correspond to different odor attributes respectively, it indicates that there are large deviations in predictions of the first preset machine learning models that output the two prediction results. When the foregoing case occurs, the prediction confidences of the first prediction results may be considered as low, and then the second preset machine learning model is introduced to assist in discrimination, to ensure the accuracy of the final prediction result.

Further, the first preset threshold may range from 8% to 12%. For example, when a difference between the probability maximum and the secondary probability maximum in the plurality of first prediction results is less than 10%, it may be considered that the two have similar deviations. In a practical application, a specific value of the first preset threshold may be adjusted according to a need.

In a specific implementation, step S106 may include the following step: determining an odor attribute corresponding to a probability maximum in the plurality of first prediction results and the second prediction results as the current odor attribute in the refrigeration appliance. Therefore, the prediction accuracy is improved through comprehensive discrimination of a plurality models.

In a specific implementation, the first preset machine learning model may include a plurality of sub-models, and different sub-models are constructed by using different algorithms. For example, the sub-model may be a candidate model trained in step S302 in the embodiment shown in FIG. 3. In this specific implementation, the first preset machine learning model is jointly constituted by a plurality of candidate models trained based on the training set.

Correspondingly, the first prediction result of the first preset machine learning model is an average of respective prediction results of the plurality of sub-models.

Similarly, the second preset machine learning model may further include a plurality of sub-models, different sub-models are constructed by using different algorithms, and the second prediction result of the second preset machine learning model is an average of respective prediction results of the plurality of sub-models.

Therefore, for a single first preset machine learning model and/or a single second preset machine learning model, an output result of the model is also a product of integrating prediction results of a plurality of sub-models, which is conducive to further improving the accuracy of the prediction result of the model.

In a specific implementation, after step S102 is performed to obtain a plurality of first prediction results, and before step S103 is performed, the odor detection method according to this embodiment may further include the following steps: determining whether all the first prediction results are higher than a second preset threshold; and obtaining, if a determination result indicates that any first prediction result is lower than the second preset threshold, updated gas data, and obtaining updated first prediction results based on the updated gas data.

Specifically, the second preset threshold can be used to represent reliability of a single first prediction result. When the reliability of the first prediction result is low, the gas data is re-acquired for the first preset machine learning model, and prediction is performed. Therefore, controlling the reliability of a plurality of first prediction results that serve as a data basis is conducive to improving the accuracy of the final prediction result.

Further, the second preset threshold may range from 55% to 65%. In a practical application, a specific value of the second preset threshold may be adjusted according to a need. For example, the second preset threshold may be 60%.

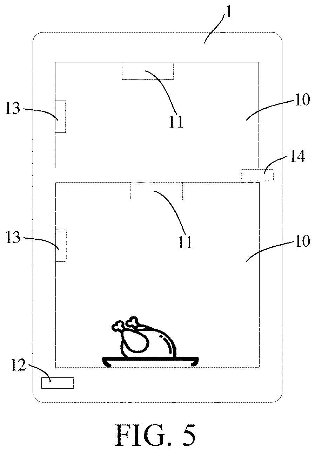

FIG. 5 is a schematic diagram of a typical application scenario of a refrigeration appliance according to an embodiment of the present invention.

Specifically, referring to FIG. 5, a refrigeration appliance 1 according to this embodiment may include: a gas detector 11, configured to acquire gas data in the refrigeration appliance 1; a control module 12, communicating with the gas detector 11 to receive the gas data, where the control module 12 is configured to perform the odor detection method according to FIG. 1 to FIG. 4, and generate a control instruction according to a current odor attribute in the refrigeration appliance 1; and a deodorization module 13, configured to adjust an operation parameter according to the control instruction in response to receiving the control instruction.

For example, the refrigeration appliance 1 may include a refrigerator, a refrigerated counter, a freezer, and the like.

Therefore, when a peculiar smell is detected based on the odor detection method provided in this implementation, the operation of the deodorization module 13 is actively controlled to eliminate the peculiar smell in time.

The gas detector 11 may be arranged in each compartment 10 of the refrigeration appliance 1 to acquire gas data in the compartment 10 in a targeted manner. Alternatively, there may be one gas detector 11, and the gas detector 11 is arranged near an air return vent of the refrigeration appliance 1 to acquire overall gas data in the refrigeration appliance 1.

The control module 12 may include a main control board of the refrigeration appliance 1. Alternatively, the control module 12 may be a module dedicated to performing the method shown in FIG. 1 to FIG. 4.

The deodorization module 13 may be arranged in each compartment 10. For any compartment 10, when a prediction result of the control module 12 on the gas data obtained by the gas detector 11 in the compartment 10 shows that a current odor attribute in the compartment 10 is unpleasant, the deodorization module 13 in the compartment 10 works to eliminate the peculiar smell in the compartment 10.

Alternatively, there may be one deodorization module 13, and the deodorization module 13 is arranged in any compartment 10 or in an air duct of the refrigeration appliance 1. When the odor attribute in any compartment 10 is determined as unpleasant, the deodorization module 13 works and achieves the overall elimination of the peculiar smell of all compartments 10 through a circulating refrigeration system in the refrigeration appliance 1.

For example, the deodorization module 13 may include an ionizer.

Communication between the gas detector 11 and the control module 12 and between the control module 12 and the deodorization module 13 may be performed in a wired or wireless manner.

Further, the refrigeration appliance 1 may further include a communication module 14. The control module 12 communicates with the gas detector 11 through the communication module 14.

Further, the control module 12 may also communicate with the deodorization module 13 through the communication module 14.

Alternatively, the communication module 14 may communicate with a device terminal of the user to inform the user of the prediction result in time. For example, the device terminal may include a mobile phone, an iPAD, a home monitoring device, and the like.

It should be noted that FIG. 5 only exemplarily shows possible arrangement positions of the control module 12, the deodorization module 13, the gas detector 11, and the communication module 14 on the refrigeration appliance 1. In a practical application, mutual position relationships between modules and specific arrangement positions on the refrigeration appliance 1 may be adjusted according to a need. The modules may be independent of each other, or may be integrated on a same chip or integrated into a same functional module. For example, the control module 12 and the communication module 14 may be integrated together.

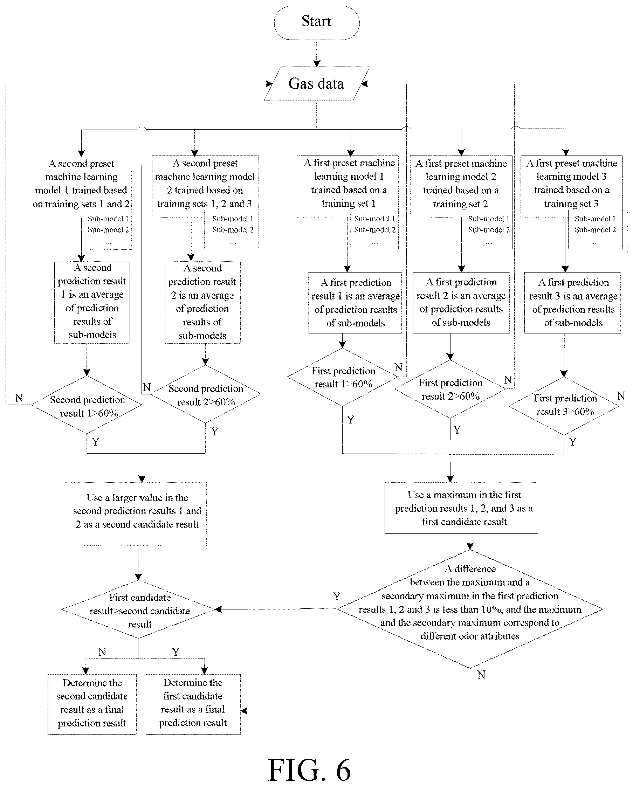

In a typical application scenario, with reference to FIG. 5 and FIG. 6, the control module 12 may store five models in advance: a first preset machine learning model 1, a first preset machine learning model 2, a first preset machine learning model 3, a second preset machine learning model 1, and a second preset machine learning model 2. The first preset machine learning model 1 is trained based on a training set 1. The first preset machine learning model 2 is trained based on a training set 2. The first preset machine learning model 3 is trained based on a training set 3. The second preset machine learning model 1 is trained based on the training set 1 and the training set 2. The second preset machine learning model 2 is trained based on the training set 1, the training set 2, and the training set 3. Each model includes a plurality of sub-models, and the sub-models are constructed based on the training set of the model by using different algorithms.

Based on the solutions according to the embodiments shown in FIG. 1 to FIG. 4, the odor detection action performed by the control module 12 may include the following process:
First, gas data acquired by the gas detector 11 is obtained.

Then, the obtained gas data is respectively inputted into the first preset machine learning model 1, the first preset machine learning model 2, the first preset machine learning model 3, the second preset machine learning model 1, and the second preset machine learning model 2. For each model, sub-models of the model perform prediction on the gas data respectively to obtain corresponding prediction results. The prediction result may include an odor attribute in the refrigeration appliance 1 and a corresponding probability.

Correspondingly, a first prediction result 1 outputted by the first preset machine learning model 1 may be an average of prediction results of sub-models included in the first preset machine learning model 1. A first prediction result 2 outputted by the first preset machine learning model 2 may be an average of prediction results of sub-models included in the first preset machine learning model 2. A first prediction result 3 outputted by the first preset machine learning model 3 may be an average of prediction results of sub-models included in the first preset machine learning model 3. A second prediction result 1 outputted by the second preset machine learning model 1 may be an average of prediction results of sub-models included in the second preset machine learning model 1. A second prediction result 2 outputted by the second preset machine learning model 2 may be an average of prediction results of sub-models included in the second preset machine learning model 2.

Next, for each prediction result, whether the prediction result is greater than 60% is determined. If the prediction result is greater than 60%, a subsequent action is further performed. Otherwise, the model that obtains the prediction result re-obtains gas data and performs prediction again to update the prediction result.

When all the prediction results are greater than 60%, a maximum in the first prediction result 1, the first prediction result 2, and the first prediction result 3 is used as a first candidate result, and a larger value in the second prediction result 1 and the second prediction result 2 is used as a second candidate result.

Simultaneously or subsequently, whether a difference between the maximum and a secondary maximum in the first prediction result 1, the first prediction result 2, and the first prediction result 3 is less than 10%, and the maximum and the secondary maximum correspond to different odor attributes respectively.

If a determination result indicates that the difference between the maximum and the secondary maximum is greater than 10%, or the difference is less than 10%, but the maximum and the secondary maximum belong to the same odor attribute, the first candidate result is determined as the final prediction result. That is, in this case, the prediction confidences of the first preset machine learning model 1, the first preset machine learning model 2, and the first preset machine learning model 3 are high, and a probability prediction maximum in the three prediction confidences is determined as the final prediction result.

If the determination result shows that the difference between the maximum and the secondary maximum is less than 10%, and the maximum and the secondary maximum respectively correspond to different odor attributes, magnitudes of the first candidate result and the second candidate result are compared, and a larger value of the two is selected as the final prediction result. That is, in this case, the prediction confidences of the first preset machine learning model 1, the first preset machine learning model 2, and the first preset machine learning model 3 are low, and the prediction results of the second preset machine learning model 1 and the second preset machine learning model 2 are introduced to assist in determining the final prediction result.

Further, if the odor attribute indicated by the final prediction result is unpleasant or extremely unpleasant, the control module 12 generates a control instruction and sends the control instruction to the deodorization module 13. In response to receiving the control instruction, the deodorization module 13 is activated to start the deodorization.

Further, the control instruction may include an operation parameter, and the deodorization module 13 performs an operation according to the operation parameter. Different odor attributes may correspond to different operation parameters. For example, an operation power in the operation parameter when the odor attribute is extremely unpleasant may be greater than an operation power when the odor attribute is unpleasant.

When the final prediction result obtained by executing the logic shown in FIG. 6 to perform the odor detection action a next time is pleasant, extremely pleasant, or neutral, the control module 12 can instruct the deodorization module 13 to stop working or switch to a low-power operating state to save energy.

Although specific implementations are described above, such implementations are not intended to limit the scope of the present disclosure, even if only a single implementation is described with respect to a specific feature. The feature example provided in the present disclosure are intended to be illustrative rather than limitative, unless otherwise stated. Therefore, the protection scope of the present invention should be subject to the scope defined by the claims.

## Claims

1. An odor detection method for a refrigeration appliance, comprising:
obtaining gas data in the refrigeration appliance;
inputting the gas data into a plurality of first preset machine learning models respectively, and obtaining first prediction results of the first preset machine learning models,
**characterized in that**
the first prediction result comprises an odor attribute in the refrigeration appliance and a corresponding probability, and the first preset machine learning model is trained based on gas data of a single type of food under a plurality of odor attributes, wherein the single type of food include by category, meat, nuts, vegetables and the like;
determining prediction confidences of the first preset machine learning models according to differences between a plurality of first prediction results; and
determining, if the prediction confidences are high, an odor attribute corresponding to a probability maximum in the plurality of first prediction results as a current odor attribute in the refrigeration appliance.

2. The odor detection method according to claim 1, **characterized by** further comprising:
inputting, if the prediction confidences are low, the gas data into at least one second preset machine learning model, and obtaining a second prediction result, wherein the second prediction result comprises an odor attribute in the refrigeration appliance and a corresponding probability, and the second preset machine learning model is trained based on gas data of mixed types of foods under a plurality of odor attributes; and
determining the current odor attribute in the refrigeration appliance according to the first prediction results and the second prediction result.

3. The odor detection method according to claim 1 or 2, **characterized in that** the determining the current odor attribute in the refrigeration appliance according to the first prediction results and the second prediction result comprises:
determining an odor attribute corresponding to a probability maximum in the plurality of first prediction results and the second prediction results as the current odor attribute in the refrigeration appliance.

4. The odor detection method according to any of the preceding claims **characterized in that** the first preset machine learning model comprises a plurality of sub-models, different sub-models are constructed by using different algorithms, and the first prediction result of the first preset machine learning model is an average of respective prediction results of the plurality of sub-models; and/or the second preset machine learning model comprises a plurality of sub-models, different sub-models are constructed by using different algorithms, and the second prediction result of the second preset machine learning model is an average of respective prediction results of the plurality of sub-models.

5. The odor detection method according to any of the preceding claims , **characterized in that** training data of the first preset machine learning model is selected from a single training set, and training data of the second preset machine learning model is selected from a plurality of training sets, wherein the training set has a one-to-one correspondence with a type of food.

6. The odor detection method according to any of the preceding claims, **characterized in that** the determining prediction confidences of the first preset machine learning models according to differences between a plurality of first prediction results comprises:
determining that the prediction confidences are low if in the plurality of first prediction results, a deviation between the probability maximum and a secondary probability maximum is less than a first preset threshold, and the probability maximum and the secondary probability maximum respectively correspond to different odor attributes; and
determining that the prediction confidences are high if in the plurality of first prediction results, the deviation between the probability maximum and the secondary probability maximum is greater than the first preset threshold.

7. The odor detection method according to any of the preceding claims, **characterized in that** the gas data comprises concentration information of at least one gas component in the refrigeration appliance.

8. The odor detection method according to any of the preceding claims, **characterized in that** before the determining prediction confidences of the first preset machine learning models according to differences between a plurality of first prediction results, the method further comprises:
determining whether all the first prediction results are higher than a second preset threshold; and
obtaining, if a determination result indicates that any first prediction result is lower than the second preset threshold, updated gas data, and obtaining updated first prediction results based on the updated gas data.

9. The odor detection method according to any of the preceding claims, **characterized in that** the odor attribute comprises at least one of the following: pleasant or unpleasant, whereby evaluation standards for odor attributes are determined according to sensitivity of a user to the odor, or according to relevant industry standards.

10. A refrigeration appliance (1), comprising:
a gas detector (11), configured to acquire gas data in the refrigeration appliance (1);
a control module (12), communicating with the gas detector (11) to receive the gas data, **characterized in that** the control module (12) is configured to perform the odor detection method according to any one of claims 1 to 8, and generate a control instruction according to a current odor attribute in the refrigeration appliance (1); and
a deodorization module (13), configured to adjust an operation parameter according to the control instruction in response to receiving the control instruction.

11. The refrigeration appliance (1) according to claim 10, **characterized by** further comprising: a communication module (14), wherein the control module (12) communicates with the gas detector (11) through the communication module (14), and/or the control module (12) communicates with the deodorization module (13) through the communication module (14).

## Patentansprüche

1. Geruchserkennungsverfahren für ein Kühlgerät, das Folgendes umfasst:
Gewinnen von Gasdaten in dem Kühlgerät,
Eingeben der Gasdaten in jeweils mehrere erste voreingestellte Maschinenlernmodelle und Gewinnen erster Prognoseergebnisse der ersten voreingestellten Maschinenlernmodelle,
**dadurch gekennzeichnet, dass**
das erste Prognoseergebnis ein Geruchsattribut in dem Kühlgerät und eine entsprechende Wahrscheinlichkeit umfasst und das erste voreingestellte Maschinenlernmodell auf der Grundlage von Gasdaten eines einzigen Lebensmitteltyps unter mehreren Geruchsattributen trainiert wird, wobei zu dem einzigen Lebensmitteltyp nach Kategorie Fleisch, Nüsse, Gemüse und dergleichen gehören,
Bestimmen von Prognosekonfidenzen für die ersten voreingestellten Maschinenlernmodelle gemäß Differenzen zwischen mehreren ersten Prognoseergebnissen und,
wenn die Prognosekonfidenzen hoch sind, Bestimmen eines Geruchsattributs, das einem Wahrscheinlichkeitsmaximum bei den mehreren ersten Prognoseergebnissen entspricht, als aktuelles Geruchsattribut im Kühlgerät.

2. Geruchserkennungsverfahren nach Anspruch 1, **dadurch gekennzeichnet, dass** es ferner Folgendes umfasst:
wenn die Prognosekonfidenzen niedrig sind, Eingeben der Gasdaten in mindestens ein zweites voreingestelltes Maschinenlernmodell und Gewinnen eines zweiten Prognoseergebnisses, wobei das zweite Prognoseergebnis ein Geruchsattribut in dem Kühlgerät und eine entsprechende Wahrscheinlichkeit umfasst und das zweite eingestellte Maschinenlernmodell auf der Grundlage von Gasdaten gemischter Lebensmitteltypen unter mehreren Geruchsattributen trainiert wird, und
Bestimmen des aktuellen Geruchsattributs im Kühlgerät gemäß den ersten Prognoseergebnissen und dem zweiten Prognoseergebnis.

3. Geruchserkennungsverfahren nach Anspruch 1 oder 2, **dadurch gekennzeichnet, dass** das Bestimmen des aktuellen Geruchsattributs im Kühlgerät gemäß den ersten Prognoseergebnissen und dem zweiten Prognoseergebnis Folgendes umfasst:
Bestimmen eines Geruchsattributs, das einem Wahrscheinlichkeitsmaximum bei den mehreren ersten und den zweiten Prognoseergebnissen entspricht, als aktuelles Geruchsattribut im Kühlgerät.

4. Geruchserkennungsverfahren nach einem der vorhergehenden Ansprüche, **dadurch gekennzeichnet, dass** das erste voreingestellte Maschinenlernmodell mehrere Teilmodelle umfasst, durch Benutzen verschiedener Algorithmen verschiedene Teilmodelle erstellt werden und es sich bei dem ersten Prognoseergebnis des ersten voreingestellten Maschinenlernmodells um einen Durchschnitt jeweiliger Prognoseergebnisse der mehreren Teilmodelle handelt und/oder dass das zweite voreingestellte Maschinenlernmodell mehrere Teilmodelle umfasst, durch Benutzen verschiedener Algorithmen verschiedene Teilmodelle erstellt werden und es sich bei dem zweiten Prognoseergebnis des zweiten voreingestellten Maschinenlernmodells um einen Durchschnitt jeweiliger Prognoseergebnisse der mehreren Teilmodelle handelt.

5. Geruchserkennungsverfahren nach einem der vorhergehenden Ansprüche, **dadurch gekennzeichnet, dass** Trainingsdaten für das erste voreingestellte Maschinenlernmodell aus einem einzigen Trainingssatz und Trainingsdaten für das zweite voreingestellte Maschinenlernmodell aus mehreren Trainingssätzen ausgewählt werden, wobei der Trainingssatz eineindeutig einem Lebensmitteltyp entspricht.

6. Geruchserkennungsverfahren nach einem der vorhergehenden Ansprüche, **dadurch gekennzeichnet, dass** das Bestimmen von Prognosekonfidenzen für die ersten voreingestellten Maschinenlernmodelle gemäß Differenzen zwischen mehreren ersten Prognoseergebnissen Folgendes umfasst:
Bestimmen, dass die Prognosekonfidenzen niedrig sind, wenn eine Abweichung zwischen dem Wahrscheinlichkeitsmaximum und einem zweiten Wahrscheinlichkeitsmaximum bei den mehreren ersten Prognoseergebnissen unter einem ersten voreingestellten Schwellenwert liegt und das Wahrscheinlichkeitsmaximum und das zweite Wahrscheinlichkeitsmaximum jeweils unterschiedlichen Geruchsattributen entsprechen, und
Bestimmen, dass die Prognosekonfidenzen hoch sind, wenn die Abweichung zwischen dem Wahrscheinlichkeitsmaximum und dem zweiten Wahrscheinlichkeitsmaximum bei den mehreren ersten Prognoseergebnissen über dem ersten voreingestellten Schwellenwert liegt.

7. Geruchserkennungsverfahren nach einem der vorhergehenden Ansprüche, **dadurch gekennzeichnet, dass** die Gasdaten Konzentrationsangaben zu mindestens einem Gasbestandteil im Kühlgerät umfassen.

8. Geruchserkennungsverfahren nach einem der vorhergehenden Ansprüche, **dadurch gekennzeichnet, dass** das Verfahren vor dem Bestimmen von Prognosekonfidenzen für die ersten voreingestellten Maschinenlernmodelle gemäß Differenzen zwischen mehreren ersten Prognoseergebnissen ferner Folgendes umfasst:
Bestimmen, ob alle ersten Prognoseergebnisse über einem zweiten voreingestellten Schwellenwert liegen, und,
wenn ein Bestimmungsergebnis anzeigt, dass ein erstes Prognoseergebnis unter dem zweiten voreingestellten Schwellenwert liegt, Gewinnen von aktualisierten Gasdaten und Gewinnen von aktualisierten ersten Prognoseergebnissen auf der Grundlage der aktualisierten Gasdaten.

9. Geruchserkennungsverfahren nach einem der vorhergehenden Ansprüche, **dadurch gekennzeichnet, dass** das Geruchsattribut mindestens eines der folgenden Attribute umfasst: angenehm oder unangenehm, wobei Bewertungsnormen für Geruchsattribute einer Empfindlichkeit eines Benutzers gegenüber dem Geruch oder relevanten Industrienormen entsprechend bestimmt werden.

10. Kühlgerät (1), das Folgendes umfasst:
einen Gasdetektor (11), der so konfiguriert ist, dass er Gasdaten in dem Kühlgerät (1) erfasst,
ein Steuermodul (12), das mit dem Gasdetektor (11) kommuniziert und so die Gasdaten empfängt,
**dadurch gekennzeichnet, dass**
das Steuermodul (12) so konfiguriert ist, dass es das Geruchserkennungsverfahren nach einem der Ansprüche 1 bis 8 durchführt und einem aktuellen Geruchsattribut in dem Kühlgerät (1) entsprechend eine Steueranweisung erzeugt, und
ein Geruchsbekämpfungsmodul (13), das so konfiguriert ist, dass es als Reaktion auf das Empfangen der Steueranweisung der Steueranweisung entsprechend einen Betriebsparameter anpasst.

11. Kühlgerät (1) nach Anspruch 10, **dadurch gekennzeichnet, dass** es ferner Folgendes umfasst: ein Kommunikationsmodul (14), wobei das Steuermodul (12) über das Kommunikationsmodul (14) mit dem Gasdetektor (11) und/oder mit dem Geruchsbekämpfungsmodul (13) kommuniziert.

## Revendications

1. Procédé de détection d'odeur pour un appareil de réfrigération, comprenant :
l'obtention de données sur le gaz dans l'appareil de réfrigération ;
l'entrée des données sur le gaz dans une pluralité de premiers modèles préréglés d'apprentissage de la machine respectivement, et l'obtention des premiers résultats de prédiction des premiers modèles préréglés d'apprentissage de la machine,
**caractérisé en ce que**
le premier résultat de prédiction comprend un attribut d'odeur dans l'appareil de réfrigération et une probabilité correspondante, et le premier modèle préréglé d'apprentissage de la machine est entraîné sur base des données sur le gaz d'un seul type d'aliment dans le cadre d'une pluralité d'attributs d'odeur, dans lequel le seul type d'aliment comprend par catégorie, de la viande, des oléagineux, des légumes et autres semblables ;
la détermination des confiances dans les prédictions des premiers modèles préréglés d'apprentissage de la machine selon des différences entre une pluralité de premiers résultats de prédiction ; et
la détermination, si les confiances dans les prédictions sont fortes, d'un attribut d'odeur correspondant à un maximum de probabilité dans la pluralité de premiers résultats de prédiction en tant qu'attribut d'odeur courant dans l'appareil de réfrigération.

2. Procédé de détection d'odeur selon la revendication 1, **caractérisé en ce qu'**il comprend en outre :
l'entrée, si les confiances de prédiction sont faibles, des données sur le gaz dans au moins un deuxième modèle préréglé d'apprentissage de la machine et l'obtention d'un deuxième résultat de prédiction, dans lequel le deuxième résultat de prédiction comprend un attribut d'odeur dans l'appareil de réfrigération et une probabilité correspondante, et le deuxième modèle préréglé d'apprentissage de la machine est entraîné sur base des données sur le gaz des types mixtes d'aliments dans le cadre d'une pluralité d'attributs d'odeur ; et
la détermination de l'attribut d'odeur courant dans l'appareil de réfrigération selon les premiers résultats de prédiction et le deuxième résultat de prédiction.

3. Procédé de détection d'odeur selon la revendication 1 ou 2, **caractérisé en ce que** la détermination de l'attribut d'odeur courant dans l'appareil de réfrigération selon les premiers résultats de prédiction et le deuxième résultat de prédiction comprend :
la détermination d'un attribut d'odeur correspondant à un maximum de probabilité dans la pluralité de premiers résultats de prédiction et des deuxièmes résultats de prédiction en tant qu'attribut d'odeur courant dans l'appareil de réfrigération.

4. Procédé de détection d'odeur selon l'une quelconque des revendications précédentes, **caractérisé en ce que** le premier modèle préréglé d'apprentissage de la machine comprend une pluralité de sous-modèles, différents sous-modèles sont construits en utilisant différents algorithmes, et le premier résultat de prédiction du premier modèle préréglé d'apprentissage de la machine est une moyenne des résultats de prédiction respectifs de la pluralité de sous-modèles ; et/ou le deuxième modèle préréglé d'apprentissage de la machine comprend une pluralité de sous-modèles, différents sous-modèles sont construits en utilisant différents algorithmes, et le deuxième résultat de prédiction du deuxième modèle préréglé d'apprentissage de la machine est une moyenne des résultats de prédiction respectifs de la pluralité de sous-modèles.

5. Procédé de détection d'odeur selon l'une quelconque des revendications précédentes, **caractérisé en ce que** les données d'entraînement du premier modèle préréglé d'apprentissage de la machine sont sélectionnées parmi un seul jeu d'entraînement, et les données d'entraînement du deuxième modèle préréglé d'apprentissage de la machine sont sélectionnées parmi une pluralité de jeux d'entraînement, dans lequel le jeu d'entraînement possède une correspondance un-un avec un type d'aliment.

6. Procédé de détection d'odeur selon l'une quelconque des revendications précédentes, **caractérisé en ce que** la détermination des confiances de prédiction des premiers modèles préréglés d'apprentissage de la machine selon des différences entre une pluralité de premiers résultats de prédiction comprend :
la détermination de ce que les confiances de prédiction sont faibles si dans la pluralité des premiers résultats de prédiction, une déviation entre le maximum de probabilité et un maximum de probabilité secondaire est inférieure à un premier seuil préréglé, et le maximum de probabilité et le maximum de probabilité secondaire correspondent respectivement à différents attributs d'odeur ; et
la détermination que les confiances de prédiction sont fortes si dans la pluralité des premiers résultats de prédiction, la déviation entre le maximum de probabilité et le maximum de probabilité secondaire est plus grande que le premier seuil préréglé.

7. Procédé de détection d'odeur selon l'une quelconque des revendications précédentes, **caractérisé en ce que** les données sur le gaz comprennent des informations de concentration d'au moins un composant du gaz dans l'appareil de réfrigération.

8. Procédé de détection d'odeur selon l'une quelconque des revendications précédentes, **caractérisé en ce que**, avant la détermination des confiances de prédiction des premiers modèles préréglés d'apprentissage de la machine selon des différences entre une pluralité des premiers résultats de prédiction, le procédé comprend en outre :
la détermination si tous les premiers résultats de prédiction sont plus forts qu'un deuxième seuil préréglé ; et
l'obtention, si un résultat de détermination indique qu'un quelconque premier résultat de prédiction est plus faible que le deuxième seuil préréglé, des données sur le gaz mises à jour, et l'obtention des premiers résultats de prédiction mis à jour sur base des données sur le gaz mises à jour.

9. Procédé de détection d'odeur selon l'une quelconque des revendications précédentes, **caractérisé en ce que** l'attribut d'odeur comprend au moins l'un des suivants : agréable ou désagréable, ce qui fait que les normes d'évaluation pour des attributs d'odeur sont déterminées en fonction de la sensibilité d'un utilisateur à l'odeur, ou selon des normes industrielles afférentes.

10. Appareil de réfrigération (1), comprenant :
un détecteur de gaz (11) configuré pour recueillir des données sur le gaz dans l'appareil de réfrigération (1) ;
un module de commande (12) communiquant avec le détecteur de gaz (11) pour recevoir les données de gaz,
**caractérisé en ce que** le module de commande (12) est configuré pour effectuer le procédé de détection d'odeur selon l'une quelconque des revendications 1 à 8, et générer une instruction de commande selon un attribut d'odeur courant dans l'appareil de réfrigération (1) ; et
un module désodorisant (13) configuré pour régler un paramètre de fonctionnement en fonction de l'instruction de commande en réponse à la réception de l'instruction de commande.

11. Appareil de réfrigération (1) selon la revendication 10, **caractérisé en ce qu'**il comprend en outre : un module de communication (14), dans lequel le module de commande (12) communique avec le détecteur de gaz (11) par le module de communication (14), et/ou le module de commande (12) communique avec le module désodorisant (13) par le module de communication (14).
